# EUROPEAN PATENT APPLICATION

(11) **EP 0 587 972 A1**
(43) Date of publication of application: **23.03.1994**
(21) Application number: 92870151.5
(22) Date of filing: 18.09.1992
(51) Int. Cl.: A23L 1/304, A23L 2/52, A23L 2/38

(54) **Sports drink without added sugar or artificial sweetener**

(71) Applicant: THE PROCTER & GAMBLE COMPANY, Cincinnati, Ohio 45202 (US)
(72) Inventor: Fischer, Christa-Maria, Dr., W-6242 Kronberg 2 (DE); Hale, Peter, W-6232 Bad Soden (DE); Lannelongue, Michel Lucien Hubert, W-6233 Kelkheim (DE); Weber, Regina Brigitte, W-6236 Eschborn (DE)
(74) Representative: Hirsch, Uwe Thomas

(57) **Abstract**

The present invention relates to beverages and in particular to sports drinks which are not sweetened by added sugar or artificial sweetener. Sports drinks in general are defined by comprising sodium, potassium, and further magnesium and/or calcium minerals to allow quick replenishing of these minerals after physical exercise. The replenishing is most conveniently and pleasantly done via a drink, thereby allowing to satisfy the need for thirst quenching. It has now has been found that the mineral replenishing via a sports drink can be provided in a better way by eliminating artificial sweeteners or added sugar in the sports drink which is typically used in commercially available sports drinks and hereto forth had not been described in the prior art.

## Description

### Field of the invention

The present invention relates to beverages and in particular to sports drinks which are not sweetened by added sugar or artificial sweetener. Sports drinks in general are defined by comprising sodium, potassium, and further magnesium and/or calcium minerals to allow quick replenishing of these minerals after physical exercise. The replenishing is most conveniently and pleasantly done via a drink, thereby allowing to satisfy the need for thirst quenching. It has now has been found that the mineral replenishing via a sports drink can be provided in a better way by eliminating artificial sweeteners or added sugar in the sports drink which is typically used in commercially available sports drinks and hereto forth had not been described in the prior art.

### Background of the invention

The prior art and commercial products available in the arena of sports drink beverages is very crowded. However no disclosure of the present invention or specific suggestion towards it has been found during a review of prior art disclosures.

In particular DE 4111040 uses potassium citrate, calcium lactate and magnesium gluconate to incorporate minerals. The need for sodium supplementation and the benefits to use only fruit juice derived sweetener is not mentioned.

EP-202106 mentions sodium supplementation for sports drinks however it requires the addition of fructose and/or aspartame both being contradictive to the present invention. EP-75114 discloses a protein containing sports drink while EP-350523 and EP-244903 both refer to compositions having a high fruit juice content but they are not directed towards sports drinks due to their lack of sodium mineral supplementation.

EP-117653, EP-301653 and EP-323667 are all directed towards beverages containing minerals, in particular calcium, potassium and magnesium, however they do not disclose the need for sodium nor are they directed towards a sports drink for mineral replenishing after exercise. EP-117653 is considered the closest prior art to the present invention. However, it is directed towards a different problem than the present invention since it attempts to improve mouthfeel characteristics and in particular allows the use of artificial sweetener. It is therefore not teaching to improve the replenishing of minerals by eliminating artificial sweetener and added sugar. Also it does not disclose the importance of sodium replenishing.

WO9112734 discloses hypotonic sports drink compositions while EP-264117, EP-40654 and AU8282214 relate to powder compositions which can be reconstituted into sports drink type beverages. Also GB1298299 discloses dry mineral food additives which are useful in the treatment of arthritis. GB1262235 also teaches to use non-fruit derived sucrose or dextrose as sweetener in beverage composition concentrates.

The problem underlying the present invention is to supply a sports drink able to provide sufficient mineral replenishing while not having draw-backs in respect to taste, after-taste or mouth feel which often is found when using artificial sweeteners or the draw-back associated with sugar( i.e. sucrose, glucose or fructose) supplementation other than from fruit juice sources used to constitute the sports drink beverage.

It is therefore an objective of the present invention to provide a mineral supplemented sports drink on the basis of all natural sweeteners in particular sugars which have not undergone other treatment than concentration and dilution in the form of a fruit juice concentrate or fruit juice.

### Summary of the invention

This and other objectives of the present invention will become more clear from the following disclosure of the invention.

The present invention relates to a sports drink which comprises supplemented sodium mineral, potassium mineral, and at least one of calcium mineral or magnesium mineral. At least half of the sodium mineral is provided as sodium citrate and/or chloride. At least half of the magnesium mineral is provided as salt of carbonate which can be solubilized in a citric and malic acid solution in particular together with calcium carbonate if present. At least half of the potassium mineral is provided as a potassium citrate which is easily dissolvable in aqueous liquid. Additionally the sports drink should comprise an ascorbic acid supplementation. The sports drink is characterized by comprising a fruit juice content which is sufficient to provide a solubilized sugar amount of 1% to 14% by weight in a single strength dilution of the sports drink. The sports drink according to the present invention may not contain artificial sweetener or added sugar derived from a different source than from the fruit juice.

A preferred sports drink according to the present invention has a pH from 2.0 to 5.0, most preferably between 3.0 and 4.5. The sugar content coming from the fruit juice to the sports drink according to the present invention is preferably between 2% and 12%, most preferably 5% to 8%, by weight of solubilized sugar. The amounts of mineral present in the sports drink of the present invention are from 0.005% to 0.1%, preferably from 0.01% to 0.03%, of sodium; from 0.01% to 0.1%, preferably from 0.02% to 0.04%, of potassium; from 0.001% to 0.15%, preferably from 0.005% to 0.015% of calcium; and from 0.001% to 0.025%, preferably from 0.003% to 0.007%, of magnesium all by weight of the single strength diluted sports drink.

The sugar contained in the sports drink is derived from a fruit juice. Fruit juices can be any of the citrus juices like orange, lemon, lime, grapefruit, tangerine juices and non-citrus juices like apple, grape, pear, cherry, berry, pineapple, peach, apricot, plum , prune, passionfruit, banana, kiwi, cashew juices as well as mixtures of these juices. Particularly preferred from the citrus juices are orange and lemon juice and from the non-citrus juices are apple and grape juice. The sugar introduced by these juices to the sports drink will usually comprise fructose and glucose. It is preferred that the weight ratio of glucose to fructose is from 0.2 to 1.4 and preferably from 0.9 to 1.1.

In addition to the sugar content desired in the sports drink of the present invention, fruit juices also introduce a flavour component to the beverage. However, for particular embodiments of the present invention, it is desirable to provide a fruit juice having a neutral or almost neutral flavour component and add natural or artificial flavours to the sports drink of the present invention in order to accommodate the desires towards particular taste sensations. It is possible to treat fruit juice to provide a neutral type flavour, in particular to reduce its acid level. In this context apple and grape juice have proven to be able to provide the desired sugar level and mild acidity without afflicting strong flavour impressions on the sports drink composition. Therefore these juice bases are particularly preferred in combination with additional flavour additives.

The sports drink according to the present invention comprises a mixture of citric and malic acid which is used to solubilize the calcium carbonate and magnesium carbonate. The amount of citric and malic acid in the present invention, whether artificially added or derived from fruit juice (including the amounts neutralized) should be no more 2% of the sports drink. The ratio citric to malic acid (including the citrate and malate), by weight as calculatable from the final sports drink composition should be from 0.1 to 20, preferably from 0.2 to 3, most preferably from 0.8 to 1.2. The total amount of acids strongly depends on additional acids comprised in the sports drink and has to be selected based on the desired tartness and pH value of the sports drink.

### Detailed description of the invention

### Definitions

As used herein the term "drinks" refers to a beverage compositions which is in a single-strength, ready-to-serve, drinkable form. Drinks of the present invention typically comprise at least 80% (preferably at least 90%) water. Drinks contemplated within the scope of the present invention include both carbonated and noncarbonated forms.

As used herein, the term "drink concentrate" refers to a drink composition in liquid form used to prepare a single strength drink. Drink concentrates within the scope of the present invention typically comprise from 30% to 70%, preferably from 40% to 60%, water. They are usually formulated to provide, drinkable beverages when diluted with 2 to 5 parts by weight water.

As used herein the term "sports drink" or "sports drink concentrate" refers to a drink or drink concentrate which comprises supplemented sodium and potassium and at least either magnesium or calcium, but preferably both. The preferred quantities of these supplemented minerals are selected to provide together with the other solubilized or dispersed compounds including the sugar from fruit juice, an isotonic or even hypertonic drink on a single strength basis. Another reason for selecting supplemented mineral amounts is to provide the most suitable replenishing of minerals for a particular physical exercise and its respective mineral depletion characteristics; in this respect all minerals contained in a sports drink composition need to be taken into consideration.

As used herein mineral amounts are referred to as the supplemented amounts. Naturally contained minerals are not included when referring to mineral amounts.

As used herein the term single strength refers to the recommended drink strength, i.e. the ready-to-serve concentration of drink compounds.

As used herein the term "fruit juice" refers to citrus juices, noncitrus juices such as apple juice, grape juice, pear juice, cherry juice, berry juice, pineapple juice, kiwi juice, cashew juice, peach juice, apricot juice, plum juice, prune juice, passion fruit juice, banana juice, and mixtures of these juices.

As used herein, the term "citrus juice" refers to fruit juices selected from orange juice, lemon juice, lime juice , grapefruit juice, tangerine juice and mixtures thereof. All amounts of fruit juice referred to herein are on the basis of 100% fruit juice in its single strength concentration.

As used herein, the term "comprising" means various components can be conjointly employed in the sports drink and sports drink concentrate of the present invention. Accordingly the term " comprising" encompasses the more restrictive terms "consisting essentially of" and "consisting of".

As used herein percentages are given as percent by weight of the sports drink in its single strength dilution. Percentages of water include the amount of water inherent in fruit juices.

### Sports drink concentrate

In the following description reference will only be made to a sports drink and its compounds. The sports drink concentrate, also contemplated by the present invention is such that upon aqueous dilution to its single strength form it is identical to the sports drink. Therefore specific recalculation of amounts, ratios, concentrations etc has not been conducted since it can easily be done by those skilled in the art. The sports drink concentrate contemplated by the present invention excludes those concentrates which cannot be reconstituted to the single strength sports drink due to solubility problems. These are easily detectable by concentrating the derived sports drink and observing at which concentration solubility problems occur. This defines the highest possible concentrate included in the present invention for any particular desired sports drink composition.

### Mineral levels, Acid System, other ingredients

To be useful in the present invention the minerals need to be "solubilized", i.e. dissolved or suspended in the sports drink or sports drink concentrate. Accordingly the amount of minerals included in the sports drink or sports drink concentrate of the present invention, will be referred to in terms of "solubilized mineral", i.e. the amount of sodium ion, calcium ion, magnesium ion, and potassium ion dissolved or suspended. The quantities of minerals solubilized in the sports drink of the present invention are quoted above. The preferred total amount of solubilized material including the acid counter ions to the mineral ions are selected such that the osmotic pressure of the sports drink is not substantially below the isotonic osmotic pressure of human blood. In particular concentrations even above the isotonic osmotic pressure are considered possible for quick replenishing of minerals for sports drinks.

The minerals according to the present invention can be supplied from a number of sources. Suitable sources for calcium magnesium and potassium include the respective salts with carbonate, sulfate, chloride, phosphate, acetate, hydrogenphosphate, dihydrogenphosphate, hydroxide as well as the respective sour salts, for example citrate, malate, gluconate or lactate. Mixtures of carbonate, citrate, chloride and, hydroxide which optionally include sulfate and preferably include gluconate are particularly preferred sources for sodium, calcium, magnesium and potassium.

The most preferred sources for each mineral are employed at a level of at least half of the mineral amount. Therefore at least half of the sodium comprised in the sports drink should come from sodium chloride or sodium citrate. At least half of the magnesium should come from magnesium carbonate. Also at least half of the potassium should be sourced from potassium citrate. For calcium no such highly preferred souring can be defined for the half amount quantity, however calcium carbonate is typically used for its particularly low taste impact.

It is well-known that sodium chloride and sodium citrate and potassium citrate are easily soluble in aqueous solutions like the fruit juice comprised in the sports drink according to the present invention. Therefore they are incorporated by simply mixing them when combining other compounds of the sports drink according to the present invention.

The solubility of calcium carbonate and magnesium carbonate is not as simple as that of sodium and potassium. Therefore the solubilizing methods described in the prior art and employing in particular citric and malic acid are used in the present invention. In particular EP-227174 and EP-244903 as well as EP-350523 can be referred to for methods to solubilize calcium in aqueous solutions. According to the present invention calcium carbonate and magnesium carbonate are solubilized in a mixture of citric and malic acid. The total amount of citric and malic acid should be below 2% of the sports drink but has to be selected depending on the desired pH of the sports drink and the tartness desired to provide a pleasant taste and mouthfeel of the sports drink.

In this context it is preferred to have a weight ratio of citric acid to malic acid in the sports drink from 0.2 to 5 preferably from 0.8 to 1.2. The amount of citric and malic acid is compounded by the presence of these acids in fruit juices which are considered when quoting the respective ratios and amounts above. In order to prevent undesired side effects of the sports drink according to the present invention, the pH of the sports drink should not fall below 2, preferably not below 3. On the other hand it has been found that some drinks having a too high pH easily develop precipitate which is highly undesirable. Also the taste desire for tartness and therefore the willingness especially of children for consumption of such a sports drink decreases drastically with too low pH values. Therefore an upper pH value of 5, preferably 4.5 should be supplied in the sports drink.

Optionally the sports drink may comprise other edible acids which include phosphoric acid, fumaric acid, adipic acid, lactic acid, tartaric acid, gluconic acid, succinic acid or their respective sour salts. Particularly preferred are edible acid systems which comprise phosphoric acid, citric acid, malic acid, gluconic acid.

High levels of chloride ion have also been found to reduce the flavour and sweetness intensity of orange juice. The reduction in aftertaste due to chloride inclusion is believed to be the result of reduced calcium-phosphate interactions due to the reduction in pH.

For sports drinks having from 0.016% to 0.35% by weight of total supplemented solubilized mineral, the level of chloride can range up to 0.09% by weight, and is preferably such that undesirable brackishness cannot be detected, i.e. no more than 0.045% by weight.

Calcium-supplemented sports drinks of the present invention can also comprise low levels of soluble phosphate. Addition of soluble phosphate, in particular phosphoric acid, at up to 0.04% has been found to improve the upfront acidity of calcium-supplemented apple juice beverages. However, this benefit does not occur when the soluble phosphate is added at much above.

Sports drinks of the present invention optionally comprise a flavour component which contains a flavour selected from fruit flavours, botanical flavours and mixtures thereof. As used herein, the term "fruit flavour" refers to those flavour derived from the reproductive part of a seed plant, especially one having a sweet pulp associated with the seed. Also included but less preferred within the term "fruit flavour" are synthetically prepared flavours made to simulate fruit flavours derived from natural sources. Particularly preferred fruit flavours are the citrus flavours including orange flavours, lemon flavours, lime flavours and grapefruit flavours. Besides citrus flavours, a variety of other fruit flavours can be used such as apple flavours, grape flavours, cherry flavours, pineapple flavours and the like. These fruit flavours can be derived from natural sources such as fruit juices and flavour oils, or else synthetically prepared.

As used herein, the term "botanical flavour" refers to flavours derived from parts of a plant or other than the fruit. As such, botanical flavours can include those flavours from nuts, bark, roots and leaves, including tea leaves. Also included within the term "botanical flavour" are synthetically prepared flavours made to simulate botanical flavours derived from natural sources. Examples of such flavours include cola flavours, tea flavours and the like. These botanical flavours can be derived from natural sources such as essential oils and extracts, or else can be synthetically prepared.

The flavour component can comprise a blend of various flavours e.g. lemon and lime flavours, cola flavours with citrus flavours to form cola flavours etc. If desired, fruit juices such as orange juice, lemon juice, lime juice, apple juice, grape juice and the like can be used in the flavour component. The flavour in the flavour component is sometimes formed into emulsion droplets which are then dispersed in the sports drink. Because these droplets usually have a specific gravity less than that of water and would therefore form a separate phase, weighting agents (which can also act as clouding agents) are typically used to keep the emulsion droplets dispersed in the sports drink. Examples of such weighting agents are brominated vegetable oils (BVO) and resin esters, in particular the ester gums. See L.F. Green, Developments in Soft Drinks Technology, Vol. 1 (Applied Science Publishers Ltd. 1978) pp. 87-93 for a further description of the use of weighting and clouding agents in liquid beverages. Besides weighting agents, emulsifiers and emulsion stabilizers can be used to stabilize the emulsion droplets. Examples of such emulsifiers and emulsion stabilizers include the gums, pectins, celluloses, polysorbates, sorbitan esters and propylene glycol alginates. See L.F. Green, supra at p. 92.

The particular amount of the flavour component effective for imparting flavour characteristics to the sports drink of the present invention ("flavour enhancing") can depend upon the flavour(s) selected, the flavour impression desired, and the form of the flavour component. For flavour components which are substantially free of fruit juice i.e. on a single strength basis, no more than about 1% fruit juice by weight of the sports drink, the flavour component can comprise at least 0.001% by weight of the sports drink and typically from 0.01% to 2% by weight especially for carbonated sports drinks. When fruit juices are used, the flavour component can comprise, on a single strength basis, up to about 40% fruit juice by weight of the sports drink, preferably from about 5% to about 15% fruit juice by weight especially for carbonated sports drinks.

Sports drinks according to the present invention may also be carbonated. Usually a sports drink will be considered to be carbonated if it comprises more than 30%, preferably more than 100% by volume of the sports drink of solubilized carbon dioxide. Carbonated beverages comprise typically from 100% to 450%, preferably from 200% to 350 % carbondioxide by volume of the sports drink.

The carbonated beverage can then be placed in a container such as a bottle or a can and then sealed. See L.F. Green, Developments in Soft Drinks Technology, Vol. 1(Applied Science Publishers Ltd. 1978), pp. 102-107, for a further description of beverage making in particular the process for carbonation.

### Fruit juice, Sweetener

A primary objective of the present invention is to provide a sports drink which is pleasing in taste, mouth-feel and other organoleptic qualities without the use of added sugar or artificial sweetener. It has long been a desire to provide a natural sports drink, however combinations of fruit juice flavours or other flavours systems apparently were never considered to satisfy the sweetness needed to make an acceptable sports drink. This is in particular considered valid since the mineral sources originally used for sports drinks - and still often in use today- had an inherent brackish, metallic or otherwise irritating taste, which was not believed to allow the use of fruit juice sweetness alone. Based on the mineral sources as indicated, the use of fruit juice as the only source of sweetness is now possible.

The sweetness provided by fruit juice, and referred to herein as sugar, is usually constituted by glucose, fructose and sucrose sugars. However exotic fruit or vegetables may contain other natural sweeteners or sweetness enhancers. Examples are those described in botanical and fruit juice literature like for examples stevioside, thaumatin and glycyrrihizin.

Juice or extracts of these exotic fruits can be included as long as no chemical treatment which would or does introduce foreign materials or cause chemical transformation is required to obtain the juice, juice concentrate or extract. It has been found that some sweeteners of exotic fruits provide a non-nutritive sweetness or sweetness enhancers which may be useful in the present invention to supplement the sweetness of the sugar of the fruit juice.

Examples of the non-nutritive sweeteners found in exotic fruit are dulcin or 4-ethoxyphenylurea from miracle fruit, (synsepalum dulcificum from the family sapotaceae) a polypeptide from serendipity berry (dioscoreophyllum cumminsii).

It has been found that the sugar amount from fruit juice in the sports drink of the present invention provides a stabilizing effect against precipitation of minerals. Also the solubilized sugar content in sports drinks has a major influence on the viscosity and hence the organoleptic experience when drinking, especially since viscosity influences the time during which the liquid remains on the tongue.

According to the invention a minimum of 1%, preferably 2%, most preferably 5% of solubilized sugar is required in the sports drink. The minimum amount of sugar depends on the other compounds of the sports drink and their impact on its viscosity, osmolarity but especially on the taste of the sports drink and on the caloric supplementation desired to be provided by the sports drink. For example when used in a hot climate a lower sugar content may be indicated since the total volume intake after and during physical exercise will be larger due to the desire to replenish lost liquid. Conversely exercise in cool conditions may require more energy supplementation while the temperature would not stir the desire for large quantities of liquid intake, so the amount of sugar solubilized in the sports drink should be higher.

The maximum amount, of solubilized sugar in the sports drink is 14%, preferably 12%, most preferably 8%.12%-14% is about the amount found in single strength orange juice. Higher amounts would result in too high viscosity and undesirable effects of long lasting sweet taste sensation which reduces or even eliminates the thirst quenching effect. It has been found that for moderate climates and typical athletic activities of non professionals 5% to 8% of solubilized sugar in the sports drink of the present invention is most desirable.

A sports drink according to the present invention therefore comprises a fruit juice amount which supplies the desired solubilized sugar quantity. For commercial reasons and taste acceptance but also for agricultural availability orange juice, lemon juice, grape juice , apple juice and mixtures thereof are best suitable to satisfy the sugar requirement while supporting the mineral solubilisation. If a flavour component is desired to be dominant in the sports drink of the present invention the fruit juice selected for supplying the required amount of solubilized sugar should have a low taste and flavour impact on the sports drink. Fruit juices can be treated to reduce their texture, flavour components and acidity in order to prepare them for use in the sports drink together with another different flavour component. Particularly preferred fruit juices for this purpose are apple juice, grape juice and mixtures thereof. They can be used together with some of the exotic fruits described above for their non-nutritive components.

Depending on the desired caloric supplementation a particular combination of sugars available from fruit juice sources can be provided. It has been found that a combination of fructose and glucose supplying a balanced supplementation of immediate and longer lasting energy can be selected. This is due to the different times it takes to make the caloric value of glucose and fructose available via the digestive system. The weight ratio of glucose to fructose should be from 0.2 to 1.4, preferably from 0.9 to 1.1.

It is theorized that higher values of the ratio of glucose to fructose favour a quickly available energy supply. The lower values favour sports drinks which are designed to provide more of the slower digested fructose and are expected to provide a longer lasting energy supply.

In addition it is believed that selecting a ratio of glucose to fructose from 0.9 to 1.1 is desirable because fructose empties faster than glucose from the stomach to the intestine while glucose is absorbed with water faster by the intestine and thereby this ratio improves rehydratation after physical exercise.

In a particularly preferred embodiment the sports drink of the present invention will comprise supplemented ascorbic acid in an amount of 0% to 0.15% and preferably from 0.02% to 0.05%. Supplemented ascorbic acid is only that which is present in the sports drink beyond that which is incorporated by the fruit juice. The fruit juice inherent ascorbic acid is not considered supplemented in the context of sports drinks according to the present invention. The source of ascorbic acid, which easily solubilizes when mixing the compounds of the sports drink, can be artificial (of industrial manufacture) or natural e.g. extracted from fruits or vegetables. The fruit juices of the present invention are substantially free of added protein. Examples of such proteins include soy protein, whey protein concentrate, and the like. These proteins can react with fruit juice aromas and flavours and, if hydrolyzed, can form short-chain peptides or amino acids which have undesirable bitter flavours. For sports drinks of the present invention, the amount of added protein is generally no more than about 0.1% by weight. Preferably, these sports drinks contain no added protein.

Other optional ingredients typically present in fruit juice can be included in the sports drinks of the present invention. For example, preservatives, vitamins and other minerals can be included. Suitable vitamins include A,D,E, B₁, B₂, B₆, B₁₂, niacin, folic acid, biotin and beta carotene. Other minerals which can be included are iron, zinc, manganese, copper and other trace minerals. If desired, natural and synthetic colorings can be included in these sports drinks.

The sports drinks described above are in particular used as liquid, mineral and energy replenishing sources during and after physical activities like sports activities. In this context the sports drinks should be formulated to prevent any overdosing of minerals. This can easily be afforded by evaluating the desirable liquid intake after a certain activity and formulating the corresponding sports drink to appropriate mineral concentrations according to the above characteristics.

### Examples :

The following examples are given to demonstrate most preferred embodiments of the present invention. They are non limiting to the scope of the invention in that sports drinks within the scope of the disclosure of the present invention are not excluded by the exemplified embodiments.

Percentages are given as weight percent of total composition. Carbonates are solubilized in an aqueous solution of the added citric and malic acid.

| Calcium and Magnesium : Premix A | | | |
|---|---|---|---|
| | Example 1 | Example 2 | Example 3 |
| Calcium carbonate¹ | 0.025% | 0.010% | 0.050% |
| Magnesium carbonate¹ | 0.020% | 0.020% | 0.050% |
| Citric acid-added¹ | 0.075% | 0.075% | 0.180% |
| Malic acid-added¹ | 0.121% | 0.121% | 0.300% |
| Water | 10% | 10% | 10% |

| | | | |
|---|---|---|---|
| ^{1.} Calcium and magnesium carbonate, citric and malic acid, sodium chloride, sodium citrate, potassium citrate and ascorbic acid are available in granular or powdered form and ought to satisfy food additive regulations when used in a sports drink for human consumption. | | | |

| Fruit juice, sodium, potassium and ascorbic acid; Premix B | | | |
|---|---|---|---|
| | Example 1 | Example 2 | Example 3 |
| FC grape white² | 10% | 3% | - |
| FC apple² | - | 4% | 15% |
| FC lemon² | 0.1% | 0.1% | 0.1% |
| Sodium chloride¹ | 0.06% | 0.030% | 0.030% |
| Sodium citrate¹ | - | - | 0.111% |
| Potassium citrate¹ | 0.079% | 0.040% | 0.100% |
| Ascorbic acid¹ | 0.028% | 0.030% | 0.050% |
| Water | 20% | 20% | 20% |

| | | | |
|---|---|---|---|
| ^{1.} Calcium and magnesium carbonate, citric and malic acid, sodium chloride, sodium citrate, potassium citrate and ascorbic acid are available in granular or powdered form and ought to satisfy food additive regulations when used in a sports drink for human consumption. | | | |
| ^{2.} FC grape white, FC apple and FC lemon are fruit juice concentrates of 45 to 72 Brix (solubilized solids, mostly sugar). They are available treated and untreated for their acidity, color, cloudiness and own flavour. They are available from many sources in many countries. The fruit juice concentrates for the examples were obtained from Böcker Company, Buxtehude, Germany. | | | |

For each example Premix A and B were prepared separately by simple mixture of the components in a household blender. The respective premix A and B were combined, then one of two flavour compositions was added and the composition was diluted with water to the solubilized sugar content as indicated below. The flavour composition contained natural and artificial flavours and suitable dyes as well as substances providing a desired cloudiness. One flavour composition was providing a lemon/lime flavour impression together with a yellow and green coloring and the other flavour composition was providing an orange/lemon flavour impression together with an orange and green colouring.

Two flavour embodiments designated lemon and orange of each example were prepared. The water used for dilution in the premixes and in the sports drinks was demineralized water. The sports drink according to the examples can also be carbonated. The mixture of premix A and premix B together with the flavour composition can also be considered as sports drink concentrates which would have to be diluted prior to consumption.

The following characteristics were found for examples 1-3 for either flavour on a single strength basis.

| | Example 1 | Example 2 | Example 3 |
|---|---|---|---|
| Fruit juice | 40% | 28% | 90% |
| Solubilized sugar | 6.2% | 4.4% | 9.6% |
| Ratio glucose to fructose | 1.0 | 0.7 | 0.4 |
| pH | 3.9 | 3.3 | 3.2 |
| ratio citric to malic (from acid and salts) | 1.023 | 0.825 | 0.305 |
| Sodium | 0.020% | 0.010% | 0.040% |
| Potassium | 0.030% | 0.015% | 0.038% |
| Calcium | 0.010% | 0.004% | 0.020% |
| Magnesium | 0.005% | 0.005% | 0.013% |
| Ascorbic acid | 0.023% | 0.025% | 0.041% |

All three examples in either flavour satisfy the objectives of the present invention. In particular example 1 was best suitable as a thirst quenching sports drink after physical exercise, showing remarkable rehydratation characteristics. The saline, brackish taste found when using artificial sweetener or added sugar was not found in taste expert tasting tests.

## Claims

1. A sports drink comprising supplemented sodium-mineral and potassium-mineral and at least one of the following calcium-mineral or magnesium-mineral,
at least half of said sodium-mineral being provided as sodium citrate, sodium chloride or mixtures thereof, at least half of said magnesium-mineral, if present, being provided as salts of carbonate, which is solubilized in an aqueous citric acid and malic acid solution, at least half of said potassium-mineral being provided as potassium citrate,
said sports drink further comprising an optional ascorbic acid supplementation of up to 0.15%, by weight of the sports drink in a single strength dilution, and
said sports drink being characterised in that
a)said sports drink comprises an amount of fruit juice sufficient to provide a solubilized sugar amount of 1% to 14% by weight of the sports drink in a single strength dilution, and
b)said sports drink does not contain artificial sweetener and does not contain sugar derived from a different source than from said fruit juice.

2. A sports drink according to claim 1 characterised in that the pH of the sports drink is from 2.0 to 5.0, preferably from 3.0 to 4.5.

3. A sports drink according to any of the preceding claims characterized in that said solubilized amount of sugar is from 2% to 12%, preferably from 5% to 8%, by weight of the sports drink in a single strength dilution.

4. A sports drink according to any of the preceding claims characterized in that said sodium-mineral is supplemented from 0.005% to 0.1% sodium by weight of said sports drink, said potassium-mineral is supplemented from 0.01% to 0.1% potassium by weight of said sports drink, said calcium-mineral is supplemented from 0.001% to 0.15% calcium by weight of said sports drink and, said magnesium-mineral is supplemented from 0.001% to 0.025% magnesium by weight of said sports drink.

5. A sports drink according to any of the preceding claims characterized in that said sodium-mineral is supplemented from 0.01% to 0.03% sodium by weight of said sports drink, said potassium-mineral is supplemented from 0.02 to 0.04% potassium by weight of said sports drink, said calcium-mineral is supplemented from 0.005% to 0.015% calcium by weight of said sports drink and said magnesium-mineral is supplemented from 0.003% to 0.007% magnesium by weight of said sports drink and ascorbic acid is supplemented from 0.02% to 0.05% by weight of said sports drink.

6. A sports drink according to any of the preceding claims characterized in that said fruit juice is apple juice, grape juice or mixtures thereof and said sports drink further comprises a flavour component.

7. A sports drink according to any of the preceding claims characterized in that said sugar amount comprises fructose and glucose, the weight ratio of glucose to fructose being from 0.2 to 1.4, preferably from 0.9 to 1.1.

8. A sports drink according to any of the preceding claims characterised in that it is carbonated, with more than 30%, preferably 100% to 450%, of CO₂ by volume of the sports drink.

9. A sports drink concentrate characterised in that it is a concentrate of a sports drink of any of the claims 1 to 8.

10. Use of the sports drink or sports drink concentrate of any of the claims 1 to 9 for mineral replenishing during or after physical activity.
